# EUROPEAN PATENT APPLICATION

(11) **EP 4 015 635 A1**
(43) Date of publication of application: **22.06.2022**
(21) Application number: 20854589.7
(22) Date of filing: 12.08.2020
(51) Int. Cl.: C12N 15/113, C12Q 1/6886, G01N 33/50

(54) **COLORECTAL CANCER DIAGNOSTIC MARKER, METHOD FOR ASSISTING DIAGNOSIS OF COLORECTAL CANCER, METHOD FOR COLLECTING DATA FOR DIAGNOSIS OF COLORECTAL CANCER, COLORECTAL CANCER DIAGNOSTIC KIT, THERAPEUTIC AGENT FOR COLORECTAL CANCER, METHOD FOR TREATING COLORECTAL CANCER, AND METHOD FOR DIAGNOSING COLORECTAL CANCER**

(30) Priority: 16.08.2019 JP 2019149338
(71) Applicant: Public University Corporation Nagoya City University, Mizuho-ku Nagoya-shi Aichi 467-8601 (JP)
(72) Inventor: SHIMURA Takaya, Nagoya-shi, Aichi 467-8601 (JP); IWASAKI Hiroyasu, Nagoya-shi, Aichi 467-8601 (JP); KATAOKA Hiromi, Nagoya-shi, Aichi 467-8601 (JP)
(74) Representative: Papula Oy
(86) International application number: PCT/JP2020/030676
(87) International publication number: WO 2021/033605

(57) **Abstract**

The present invention provides a colorectal cancer diagnostic marker which can determine the presence or absence of colorectal cancer with high accuracy and which can also detect early-stage colorectal cancer with high sensitivity; a method which is for assisting the diagnosis of colorectal cancer, and measures an expression level of the colorectal cancer diagnostic marker; a method for collecting data for diagnosis of colorectal cancer; a method for diagnosing colorectal cancer; a method for treating colorectal cancer; a colorectal cancer diagnostic kit including a primer that is specific to the colorectal cancer diagnostic marker; and a therapeutic agent for colorectal cancer containing an inhibitor of the colorectal cancer diagnostic marker.

The colorectal cancer diagnostic marker of the present invention is at least one microRNA selected from a group consisting of hsa-miR-129-1-3p, hsa-miR-566, and hsa-miR-598-5p.

## Description

### [Technical Field]

The present invention relates to a colorectal cancer diagnostic marker, a method for assisting diagnosis of colorectal cancer, a method for collecting data for diagnosis of colorectal cancer, a colorectal cancer diagnostic kit, a therapeutic agent for colorectal cancer, a method for diagnosing colorectal cancer, and a method for treating colorectal cancer.

Priority is claimed on Japanese Patent Application No. 2019-149338, filed August 16, 2019, the content of which is incorporated herein by reference.

### [Background Art]

A total colonoscopy and a fecal occult blood test are known as methods for examining the presence or absence of colorectal cancer (for example, Non-Patent Documents 1 and 2). The total colonoscopy requires a long time, is highly invasive, and is expensive. Therefore, the total colonoscopy is not recommended for medical examination for healthy subjects and screening tests.

For this reason, the fecal occult blood test is often carried out in the screening of colorectal cancer.

In addition, a carcinoembryonic antigen (CEA) and a carbohydrate antigen 19-9 (CA19-9) are known as biomarkers for colorectal cancer.

### [Citation List]

### [Non-Patent Documents]

[Non-Patent Document 1]
   National Cancer Center "Cancer Information Service" (URL: https://ganjoho.jp/med_pro/pre_scr/screening/screening_colon.html, access date: July 12, 2019)
[Non-Patent Document 2]
   "New Medical World Weekly, No. 2940, August 08, 2011" published by Igaku-Shoin Ltd. (URL: http://www.igaku-shoin.co.jp/paperDetail.do?id=PA02940_05, access date: July 12, 2019)

### [Summary of Invention]

### [Technical Problem]

However, a fecal occult blood reaction has a high probability of misidentifying a healthy subject as suffering from colorectal cancer, that is, a high probability of false positives. Therefore, the accuracy at determining the presence or absence of colorectal cancer is insufficient in the fecal occult blood reaction.

In addition, Non-Patent Document 2 describes that a detection rate of colorectal cancer by an immunological occult blood reaction is 30% to 40% in early-stage colorectal cancer. As described above, there is a problem in that the detection sensitivity is extremely low even in actual clinical practice.

Even with conventional biomarkers such as CEA and CA19-9, the detection rate of colorectal cancer is low and the detection sensitivity is not sufficient.

The present invention provides a colorectal cancer diagnostic marker which enables highly accurate determination of the presence or absence of colorectal cancer and high-sensitivity detection of early-stage colorectal cancer.

### [Solution to Problem]

A first aspect of the present invention has the aspects described in the following [1] to [13].
[1] A colorectal cancer diagnostic marker, comprising: at least one microRNA selected from a group consisting of hsa-miR-129-1-3p, hsa-miR-566, and hsa-miR-598-5p.
[2] The colorectal cancer diagnostic marker according to [1], in which the microRNA is a combination of hsa-miR-129-1-3p and hsa-miR-566.
[3] The colorectal cancer diagnostic marker according to [1] or [2], in which the marker is contained in a human body fluid.
[4] A method for assisting diagnosis of colorectal cancer, including measuring an expression level of the colorectal cancer diagnostic marker according to any one of [1] to [3] in a sample collected from a test object, and comparing the expression level with a reference value.
[5] The method according to [4], in which the expression level is standardized using the following miRNA in the sample as a standardization factor in order to compare the expression level with the reference value.
   miRNA: either or both of hsa-miR-4669 and hsa-miR-6756-5p.
[6] The method according to [4] or [5], in which a degree of progression of the colorectal cancer is stage 0 or stage I.
[7] A method for collecting data for diagnosis of colorectal cancer, including measuring an expression level of the colorectal cancer diagnostic marker according to any one of [1] to [3] in a sample collected from a test object.
[8] The method according to [7], in which, after measuring the expression level, the expression level is standardized using the following miRNA in the sample as a standardization factor.
   miRNA: either or both of hsa-miR-4669 and hsa-miR-6756-5p.
[9] The method according to [7] or [8], in which a degree of progression of the colorectal cancer is stage 0 or stage I.
[10] A colorectal cancer diagnostic kit for use in diagnosis of colorectal cancer, including a primer that is specific to the colorectal cancer diagnostic marker according to any one of [1] to [3].
[11] The colorectal cancer diagnostic kit according to [10], further including at least one selected from the group consisting of an extraction reagent for extracting the microRNA from a sample, a cDNA synthesis reagent for synthesizing cDNA of the microRNA, a sample collection container, a primer for standardization that is specific to miRNA as a standardization factor, a reverse transcriptase, a DNA polymerase, and an instruction manual.
[12] The colorectal cancer diagnostic kit according to [10] or [11], in which a degree of progression of the colorectal cancer is stage 0 or stage I.
[13] A therapeutic agent for colorectal cancer, including an inhibitor of at least one microRNA selected from a group consisting of hsa-miR-129-1-3p, hsa-miR-566, and hsa-miR-598-5p.

A second aspect of the present invention further has the aspects described in <<1>> to <<23>> below in addition to the aspects described in the above <<1>> to <<13>>.
<<1>> A colorectal cancer diagnostic marker, comprising: at least one microRNA selected from a group consisting of hsa-miR-129-1-3p, hsa-miR-566, and hsa-miR-598-5p.
«2» The colorectal cancer diagnostic marker according to <<1>>, in which the microRNA is a combination of hsa-miR-129-1-3p and hsa-miR-566.
«3» The colorectal cancer diagnostic marker according to <<1>> or <<2>>, in which the marker is contained in a human body fluid.
«4» A method for diagnosing colorectal cancer, including measuring an expression level of the colorectal cancer diagnostic marker according to any one of <<1>> to «3» in a sample collected from a test object, and comparing the expression level with a reference value.
«5» The method for diagnosing colorectal cancer according to <<4>>, in which the expression level is standardized using miRNA in the sample as a standardization factor in order to compare the expression level with the reference value.
«6» The method for diagnosing colorectal cancer according to <<5>>, in which the miRNA is either or both of hsa-miR-4669 and hsa-miR-6756-5p.
«7» The method for diagnosing colorectal cancer according to any one of «4» to <<6>>, in which a degree of progression of the colorectal cancer is stage 0 or stage I.
<<8>> A method for treating colorectal cancer, including diagnosing a test object using the method for diagnosing colorectal cancer according to any one of <<4>> to <<7>>, and administering a therapeutic agent for colorectal cancer to the test object in a case where the test object is diagnosed as suffering from colorectal cancer.
<<9>> A method for treating colorectal cancer, including diagnosing a test object using the method for diagnosing colorectal cancer according to any one of <<4>> to <<7>>, and carrying out a surgical procedure on the test object in a case where the test object is diagnosed as suffering from colorectal cancer.
«10» A method for determining colorectal cancer, which is a method for determining the presence or absence of colorectal cancer, including measuring an expression level of the colorectal cancer diagnostic marker according to any one of <<1>> to <<3>> in a sample collected from a test object, and comparing the expression level with a reference value.
«11» The method for determining colorectal cancer according to «10», in which the expression level is standardized using miRNA in the sample as a standardization factor in order to compare the expression level with the reference value.
«12» The method for determining colorectal cancer according to «11», in which the miRNA is either or both of hsa-miR-4669 and hsa-miR-6756-5p.
<<13>> The method for determining colorectal cancer according to any one of <<10>> to <<12>>, in which a degree of progression of the colorectal cancer is stage 0 or stage I.
<<14>> A method for treating colorectal cancer, including determining the presence or absence of colorectal cancer in a test object using the method for determining colorectal cancer according to any one of <<10>> to <<13>>, and administering a therapeutic agent for colorectal cancer to the test object in a case where the test object is determined to suffer from colorectal cancer.
«15» A method for treating colorectal cancer, including determining the presence or absence of colorectal cancer in a test object using the method for determining colorectal cancer according to any one of <<10>> to <<13>>, and carrying out a surgical procedure on the test object in a case where the test object is determined to suffer from colorectal cancer.
«16» A method for examining colorectal cancer, which is a method for examining the presence or absence of colorectal cancer, including measuring an expression level of the colorectal cancer diagnostic marker according to any one of <<1>> to <<3>> in a sample collected from a test object, and comparing the expression level with a reference value.
«17» The method for examining colorectal cancer according to «16», in which the expression level is standardized using miRNA in the sample as a standardization factor in order to compare the expression level with the reference value.
«18» The method for examining colorectal cancer according to «17», in which the miRNA is either or both of hsa-miR-4669 and hsa-miR-6756-5p.
<<19>> The method for examining colorectal cancer according to any one of <<16>> to <<18>>, in which a degree of progression of the colorectal cancer is stage 0 or stage I.
<<20>> A method for treating colorectal cancer, including examining the presence or absence of colorectal cancer in a test object using the method for examining colorectal cancer according to any one of <<16>> to <<19>>, and administering a therapeutic agent for colorectal cancer to the test object in a case where the test object is determined to suffer from colorectal cancer.
«21» A method for treating colorectal cancer, including examining the presence or absence of colorectal cancer in a test object using the method for examining colorectal cancer according to any one of <<16>> to <<19>>, and carrying out a surgical procedure on the test object in a case where the test object is determined to suffer from colorectal cancer.
<<22>> A therapeutic agent for colorectal cancer, including an inhibitor of at least one microRNA selected from a group consisting of hsa-miR-129-1-3p, hsa-miR-566, and hsa-miR-598-5p.
<<23>> A method for treating colorectal cancer, including inhibiting at least one microRNA selected from a group consisting of hsa-miR-129-1-3p, hsa-miR-566, and hsa-miR-598-5p of a test object.

### [Effects of Invention]

According to the present invention, a colorectal cancer diagnostic marker is provided which enables highly accurate determination of the presence or absence of colorectal cancer and high-sensitivity detection of early-stage colorectal cancer.

### [Brief Description of Drawings]

Fig. 1 is a view showing an outline of verification of Examples.
Fig. 2 is a view showing the results of miRNA array analysis in cohort 1.
Fig. 3 is a view showing ROC curves of individual colorectal cancer diagnostic markers in cohort 2.
Fig. 4 is a view showing ROC curves of individual colorectal cancer diagnostic markers in cohort 3.
Fig. 5 is a view showing a comparison of expression levels of hsa-miR-129-1-3p in cohort 4 between a healthy subject and a stage 0 or stage I colorectal cancer patient.
Fig. 6 is a view showing a comparison of expression levels of hsa-miR-566 in cohort 4 between a healthy subject and a stage 0 or stage I colorectal cancer patient.
Fig. 7 is a view showing ROC curves of individual colorectal cancer diagnostic markers in cohort 4.
Fig. 8 is a view showing a comparison of expression levels of hsa-miR-129-1-3p in normal tissue and colorectal cancer tissue in analysis using formalin-fixed paraffin embedding.
Fig. 9 is a view showing a comparison of expression levels of hsa-miR-566 in normal tissue and colorectal cancer tissue in analysis using formalin-fixed paraffin embedding.
Fig. 10 is a view in which an expression level (2^{-ΔCt}) of hsa-miR-129-1-3p and an expression level (2^{-ΔCt}) of hsa-miR-566 in (i) exosomes in a culture solution, (ii) a culture solution, and (iii) a cell body, for SW480 and HCT116, are plotted on a vertical axis and a horizontal axis, respectively.
Fig. 11 is a photograph used for evaluation of cell motility of SW480 and HCT116.
Fig. 12 is a graph used for evaluation of cell motility of SW480 and HCT116.
Fig. 13 is a view showing the results of gene ontology analysis.
Fig. 14 is a view showing the results of gene ontology analysis.

### [Description of Embodiments]

The meanings of the following terms in the present specification and claims are as described below.

The "hsa-miR-129-1-3p" is the hsa-miR-129-1-3p gene (miRBase Accession No. MIMAT0004548) set forth in SEQ ID NO: 1, which includes homologs or orthologs of non-human species.

The "hsa-miR-566" is the hsa-miR-566 gene (miRBase Accession No. MIMAT0003230) set forth in SEQ ID NO: 2, which includes homologs or orthologs of non-human species.

The "hsa-miR-598-5p" is the hsa-miR-598-5p gene (miRBase Accession No. MIMAT0026620) set forth in SEQ ID NO: 3, which includes homologs or orthologs of non-human species.

The "hsa-miR-4669" is the hsa-miR-4669 gene (miRBase Accession No. MIMAT0019749) set forth in SEQ ID NO: 4, which includes homologs or orthologs of non-human species.

The "hsa-miR-6756-5p" is the hsa-miR-6756-5p gene (miRBase Accession No. MIMAT0027412) set forth in SEQ ID NO: 5, which includes homologs or orthologs of non-human species.

The term "primer" means a nucleotide that forms a complementary pair with either or both of DNA and RNA.

The meanings of the following terms in the present specification are as described below.

The term "variant" means a naturally occurring variant due to polymorphism, mutation, or the like; or a variant containing deletion, substitution, addition, or insertion of one or more bases.

The term "derivative" means a labeled derivative with a fluorescent group, a radioactive isotope, or the like; a modified nucleotide having an organic functional group; a nucleotide that has undergone base rearrangement, double bond saturation, deamination, substitution of an oxygen molecule with a sulfur molecule, or the like; or the like. However, the derivative is not limited to these examples.

The term "test object" means a primate such as a human or a chimpanzee; a mammal such as a rodent, for example, a mouse or a rat; a pet animal such as a dog or a cat; or a domestic animal such as a cow, a horse, a sheep, or a goat.

The term "subject" means a human as the test object.

The terms "healthy object" and "healthy subject" mean a living organism that is a test object and does not suffer from colorectal cancer.

The term "accuracy" means a value of ((number of true positives) + (number of true negatives))/(total number of specimen materials).

The term "sensitivity" means a value of (number of true positives)/((number of true positives) + (number of false negatives)). Higher sensitivity makes it easier to detect colorectal cancer at an early stage, which contributes to complete resection of an affected area of the cancer and a reduction of recurrence rate.

The term "specificity" means a value of (number of true negatives)/((number of true negatives) + (number of false positives)). Higher specificity makes it easier to prevent a healthy object from being mistakenly identified as a colorectal cancer patient, and therefore prevents unnecessary additional tests from being carried out, which contribute to reducing the burden on the patients and reducing medical costs.

The act specified by each of the terms "test," "evaluation," and "examination" does not include a medical practice (for example, an act of diagnosing a human illness, or an act of treating a human illness) by a physician in Japan and in countries where the medical practice is excluded from the subject of the patent.

The term "to" indicating a numerical range means that the numerical values described before and after "to" are included as a lower limit value and an upper limit value, respectively.

### <Colorectal cancer diagnostic marker>

The colorectal cancer diagnostic marker of the present invention is at least one microRNA selected from a group consisting of hsa-miR-129-1-3p, hsa-miR-566, and hsa-miR-598-5p. That is, the colorectal cancer diagnostic marker of the present invention is one microRNA selected from a group consisting of hsa-miR-129-1-3p, hsa-miR-566, and hsa-miR-598-5p, or is a combination of two or more microRNAs selected from a group consisting of hsa-miR-129-1-3p, hsa-miR-566, and hsa-miR-598-5p.

The microRNA may further include at least one or more selected from the group consisting of a variant and a derivative of hsa-miR-129-1-3p, a variant and a derivative of hsa-miR-566, and a variant and a derivative of hsa-miR-598-5p as long as the effects of the present invention are not impaired.

The combination of microRNAs is preferably a combination of hsa-miR-129-1-3p and hsa-miR-566 from the viewpoint of further improving the accuracy, sensitivity, and specificity in determining the presence or absence of colorectal cancer. In addition, in a case where the combination of microRNAs is a combination of hsa-miR-129-1-3p and hsa-miR-566, a significant effect of excellent sensitivity and specificity in determining the presence or absence of colorectal cancer can be obtained even in a case where the colorectal cancer is stage 0 or stage I.

hsa-miR-129-1-3p can be specified, for example, by the method described in RNA. 9: 175-179 (2003). hsa-miR-129-1 stem-loop (miRBase Accession No. MI0000252, SEQ ID NO: 6) is known as a precursor of hsa-miR-129-1-3p. The hsa-miR-129-1 stem-loop has a stem-loop structure.

hsa-miR-566 can be specified, for example, by the method described in Proc Natl Acad Sci USA. 103: 3687-3692 (2006). hsa-miR-566 stem-loop (miRBase Accession No. MI0003572, SEQ ID NO: 7) is known as a precursor of hsa-miR-566. The hsa-miR-566 stem-loop has a stem-loop structure.

hsa-miR-598-5p can be specified, for example, by the method described in Proc Natl Acad Sci USA. 103: 3687-3692 (2006). hsa-miR-598 stem-loop (miRBase Accession No. MI0003610, SEQ ID NO: 8) is known as a precursor of hsa-miR-598-5p. The hsa-miR-598-5p stem-loop has a stem-loop structure.

The colorectal cancer diagnostic marker of the present invention is preferably contained in a human body fluid. In a case where the colorectal cancer diagnostic marker is contained in a human body fluid, the test is non-invasive and convenient. In addition, there is an advantage in that the presence or absence of colorectal cancer can be tested at places other than medical institutions such as homes.

Specific examples of the body fluid include body fluids such as blood, milk, urine, saliva, lymph, cerebrospinal fluid, amniotic fluid, tears, sweat, rhinorrhea, and stool juice. However, the body fluid is not limited to these examples.

Among these, urine is preferable as the body fluid from the viewpoint that it is easy to collect. The body fluid may be a treatment liquid that has undergone a pretreatment such as removing unnecessary components from each of the above-mentioned body fluids; or a culture solution obtained by culturing cells contained in each of the above-mentioned body fluids.

### (Mechanism of action)

The colorectal cancer diagnostic marker of the present invention described above is significantly highly expressed in colorectal cancer patients as compared with healthy subjects, as shown in Examples described later. Therefore, it is possible to determine whether or not a test object suffers from colorectal cancer by measuring an expression level of the colorectal cancer diagnostic marker in a sample collected from the test object and comparing the measured value with a reference value to evaluate the magnitude.

In addition, the colorectal cancer diagnostic marker of the present invention tends to be significantly highly expressed even in an early stage 0 or stage I colorectal cancer patient group, as shown in Examples described later. Therefore, according to the colorectal cancer diagnostic marker of the present invention, the presence or absence of colorectal cancer can be determined with excellent sensitivity and specificity even in a case where the degree of progression of colorectal cancer is stage 0 or stage I.

### (Uses)

The colorectal cancer diagnostic marker of the present invention described above can be applied to, for example, a method for assisting diagnosis of colorectal cancer, a method for evaluating the possibility of suffering from colorectal cancer, a method for diagnosis of colorectal cancer, a method for testing colorectal cancer, a method for examining the possibility of suffering from colorectal cancer, a method for collecting data for diagnosis of colorectal cancer, an in vitro method for assisting diagnosis of colorectal cancer, and a colorectal cancer diagnostic kit, each of which will be described later.

In addition to the above-mentioned uses, the colorectal cancer diagnostic marker of the present invention can also be applied to a method for diagnosing colorectal cancer, a method for determining colorectal cancer, a method for examining colorectal cancer, and a method for treating colorectal cancer.

The details of the uses of the colorectal cancer diagnostic marker of the invention will be described in detail later in the section of <Method for assisting diagnosis of colorectal cancer>.

### <Method for assisting diagnosis of colorectal cancer>

The method for assisting diagnosis of colorectal cancer of the present invention provides information for making a decision which assists the diagnosis of whether or not a test object suffers from colorectal cancer. A diagnosis of colorectal cancer may be made based on the information for making a decision.

In the method for assisting diagnosis of colorectal cancer of the present invention, an expression level of the colorectal cancer diagnostic marker of the present invention in a sample collected from a test object is measured. Next, the expression level of the colorectal cancer diagnostic marker is compared with a reference value.

The method for collecting a sample from the test object is not particularly limited. For example, the method for collecting a sample is not particularly limited as long as it is a method capable of collecting various body fluids described in <Colorectal cancer diagnostic marker>. For example, the method used in common urinalysis may be used in a case where urine is used as the body fluid. The method used in common tests may also be used in a case where other body fluids are used.

As shown in Examples described later, the inventors of the present invention have found that a specific microRNA, which is the colorectal cancer diagnostic marker of the present invention, is significantly highly expressed in colorectal cancer patients as compared with healthy subjects.

Therefore, it is possible to detect colorectal cancer and determine whether or not the test object suffers from colorectal cancer by evaluating whether or not the expression level of the colorectal cancer diagnostic marker of the present invention is higher than a certain reference value.

The expression level of the colorectal cancer diagnostic marker can be measured by, for example, quantitative reverse transcription (RT)-PCR.

The quantitative RT-PCR of the colorectal cancer diagnostic marker can be carried out, for example, according to the following method (1).
- Method (1): A reverse transcription reaction of a colorectal cancer diagnostic marker is carried out, and then quantitative PCR is carried out using template DNA (1) obtained by the reverse transcription reaction.

In the method (1), first, reverse transcription of microRNA in a sample collected from a test object, that is, a colorectal cancer diagnostic marker is carried out. Here, the microRNA is at least one selected from a group consisting of hsa-miR-129-1-3p, hsa-miR-566, and hsa-miR-598-5p. These microRNAs are mature microRNAs (mature miRNAs) that have undergone processing.

Therefore, it is preferable to use the following primer A1 in the reverse transcription of the colorectal cancer diagnostic marker.
- Primer A1: a looped RT primer specific for the colorectal cancer diagnostic marker.

The primer A1 is not particularly limited as long as it specifically binds to at least one selected from a group consisting of hsa-miR-129-1-3p, hsa-miR-566, and hsa-miR-598-5p to form a stem-loop structure.

The primer A1 has a loop structure. The binding of the primer A1 to the colorectal cancer diagnostic marker forms a stem-loop structure. Reverse transcriptase recognizes the stem-loop structure, and therefore the reverse transcription reaction of the colorectal cancer diagnostic marker proceeds. The template DNA (1) of the colorectal cancer diagnostic marker is synthesized by the reverse transcription reaction.

The reverse transcriptase used for the reverse transcription reaction is not particularly limited. Any reverse transcriptase commonly used in the laboratory can be used. A recombinant protein may be used as the reverse transcriptase.

Next, quantitative PCR is carried out using the template DNA (1) of the colorectal cancer diagnostic marker obtained by the reverse transcription reaction of the colorectal cancer diagnostic marker. In quantitative PCR, it is preferable to use the following two types of primer A2 and primer A3.
- Primer A2: a forward primer that specifically binds to the template DNA (1).
- Primer A3: a reverse primer that specifically binds to the template DNA (1). By carrying out quantitative PCR using these primers A2 and A3, the expression level of the colorectal cancer diagnostic marker can be measured from the template DNA (1) of the colorectal cancer diagnostic marker.

The DNA polymerase used for quantitative PCR is not particularly limited. Any DNA polymerase commonly used in the laboratory can be used. A recombinant protein may be used as the DNA polymerase.

The primer A1, the primer A2, and the primer A3 can be prepared by common nucleic acid synthesis and nucleotide synthesis. For example, the primers can be obtained from a company that undertakes contract synthesis of nucleic acids. In addition, TaqMan Advanced MicroRNA Assay (available from Thermo Fisher Scientific, USA) can also be used as a commercially available kit for detecting the colorectal cancer diagnostic marker. Therefore, a person skilled in the art can obtain the primer A1, the primer A2, and the primer A3 by a conventional method without undue burden, and therefore can examine the expression level of the colorectal cancer diagnostic marker.

For example, the catalog number of the TaqMan Advanced MicroRNA Assay containing the following primer A11, primer A21, and primer A31 is A25576, and its Assay ID is 480873_mir.
- Primer A11: a looped RT primer that specifically binds to hsa-miR-129-1-3p.
- Primer A21: a forward primer that specifically binds to the template DNA (1) of hsa-miR-129-1-3p.
- Primer A31: a reverse primer that specifically binds to the template DNA (1) of hsa-miR-129-1-3p.

For example, the catalog number of the TaqMan Advanced MicroRNA Assay containing the following primer A12, primer A22, and primer A32 is A25576, and its Assay ID is 479373_mir.
- Primer A12: a looped RT primer that specifically binds to hsa-miR-566.
- Primer A22: a forward primer that specifically binds to the template DNA (1) of hsa-miR-566.
- Primer A32: a reverse primer that specifically binds to the template DNA (1) of hsa-miR-566.

For example, the catalog number of the TaqMan Advanced MicroRNA Assay containing the following primer A13, primer A23, and primer A33 is A25576, and its Assay ID is 479080_mir.
- Primer A13: a looped RT primer that specifically binds to hsa-miR-598-5p.
- Primer A23: a forward primer that specifically binds to the template DNA (1) of hsa-miR-598-5p.
- Primer A33: a reverse primer that specifically binds to the template DNA (1) of hsa-miR-598-5p.

In the method for assisting diagnosis of colorectal cancer of the present invention, the expression level of microRNA can be calculated based on a cutoff value of the number of cycles in quantitative PCR.

In quantitative PCR, in a case where an amount of nucleic acid to be measured is relatively large in a sample, the number of cycles until an amplified amount of nucleic acid reaches a certain value is relatively short. On the other hand, in a case where an amount of nucleic acid to be measured is relatively small in a sample, the number of cycles until an amplified amount of nucleic acid reaches a certain value becomes long, and therefore a cutoff value becomes large. The amount of nucleic acid to be measured is calculated by a calibration curve, with the number of cycles until the amplified amount of this nucleic acid reaches a certain threshold value as a cutoff value.

The threshold value of the amplified amount of nucleic acid involved in determining the cutoff value can be appropriately set according to conditions such as the age and sex of the test object, the total amount of RNA in the sample to be collected, the type of colorectal cancer diagnostic marker to be used, the collection method, and the type of specimen material.

In the method for assisting diagnosis of colorectal cancer of the present invention, it is preferable to standardize the expression level of the colorectal cancer diagnostic marker using miRNA in the sample as a standardization factor, in order to compare the expression level of the colorectal cancer diagnostic marker with the reference value. By standardizing the expression level of the colorectal cancer diagnostic marker, the relative magnitude of the expression level of the colorectal cancer diagnostic marker can be determined more accurately, and the presence or absence of colorectal cancer can be determined with higher accuracy.

The miRNA used as the standardization factor is preferably either or both of hsa-miR-4669 and hsa-miR-6756-5p and more preferably a combination of hsa-miR-4669 and hsa-miR-6756-5p.

The inventors of the present invention have found that hsa-miR-4669 and hsa-miR-6756-5p are constantly expressed in the urine of species of organism such as humans, and the expression levels thereof are stable. hsa-miR-4669 and hsa-miR-6756-5p exhibit stable expression levels as described above and are therefore useful as a standardization factor for the colorectal cancer diagnostic marker of the present invention contained in a body fluid collected from a test object.

In addition, hsa-miR-4669 and hsa-miR-6756-5p are also useful as a standardization factor for any miRNA contained in a body fluid collected from a test object.

hsa-miR-4669 and hsa-miR-6756-5p as the standardization factor may further include at least one or more selected from the group consisting of a variant and a derivative of hsa-miR-4669, and a variant and a derivative of hsa-miR-6756-5p as long as the effects of the present invention are not impaired.

hsa-miR-4669 can be specified, for example, by the method described in Cancer Res. 71: 78-86 (2011). hsa-miR-4669 stem-loop (miRBase Accession No. MI0017300, SEQ ID NO: 9) is known as a precursor of hsa-miR-4669. The hsa-miR-4669 stem-loop has a stem-loop structure.
hsa-miR-6756-5p can be specified, for example, by the method described in Genome Res. 22: 1634-1645 (2012). hsa-miR-6756 stem-loop (miRBase Accession No. MI0022601, SEQ ID NO: 10) is known as a precursor of hsa-miR-6756-5p. The hsa-miR-6756 stem-loop has a stem-loop structure.

In a case of standardizing an expression level of a colorectal cancer diagnostic marker using miRNA in a sample as the standardization factor in the method for assisting diagnosis of colorectal cancer of the present invention, the expression level of the colorectal cancer diagnostic marker may be calculated based on the following ΔCt. By using ΔCt, the expression level of the colorectal cancer diagnostic marker can be quantified as 2^{-ΔCt}.
ΔCt: a value obtained by subtracting the cutoff value of the standardization factor from the cutoff value of the colorectal cancer diagnostic marker.

By using the ΔCt value, the ΔCt value can be used for evaluating the expression level of the colorectal cancer diagnostic marker in each specimen material. Specifically, whether or not a test object suffers from colorectal cancer can be easily determined by using 2^{-ΔCt} as a measured value of the expression level of each colorectal cancer diagnostic marker and comparing 2^{-ΔCt} with a reference value.

Here, in a colorectal cancer patient, the colorectal cancer diagnostic marker is relatively highly expressed, and therefore the -ΔCt value is relatively large and the 2^{-ΔCt} value is relatively large. On the other hand, in a healthy object, the -ΔCt value is relatively small and the 2^{-ΔCt} value is relatively small.

In this case, 2^{-ΔCt} in a healthy object can be used as a reference value, in a case where ΔCt, which is an index in a healthy object, is known.

The cutoff value of the standardization factor in the ΔCt value can be an average value of cutoff values of hsa-miR-4669 and hsa-miR-6756-5p in a sample collected from a test object.

The expression level of miRNA used as the standardization factor can be measured, for example, by quantitative PCR. Specifically, for example, the expression level of each of hsa-miR-4669 and hsa-miR-6756-5p can be calculated based on the cutoff value of the number of cycles in the quantitative PCR of the standardization factor.

The quantitative PCR of the standardization factor can be carried out, for example, according to the following method (2).
- Method (2): A reverse transcription reaction of a standardization factor is carried out, and then quantitative PCR is carried out using template DNA (2) obtained by the reverse transcription reaction.

In the method (2), first, reverse transcription of miRNA in a sample collected from a test object, that is, a standardization factor is carried out. Here, the miRNA is at least one selected from a group consisting of hsa-miR-4669 and hsa-miR-6756-5p. These miRNAs are mature miRNAs that have undergone processing.

Therefore, it is preferable to use the following primer B1 in the reverse transcription of the standardization factor.
- Primer B1: a looped RT primer specific for the standardization factor.

The primer B1 is not particularly limited as long as it specifically binds to at least one selected from a group consisting of hsa-miR-4669 and hsa-miR-6756-5p to form a stem-loop structure.

The primer B1 has a loop structure. Binding of the primer B1 to the standardization factor forms a stem-loop structure. Reverse transcriptase recognizes the stem-loop structure, and therefore the reverse transcription reaction of the standardization factor proceeds. The template DNA (2) of the standardization factor is synthesized by the reverse transcription reaction.

The reverse transcriptase used for the reverse transcription reaction is not particularly limited. Any reverse transcriptase commonly used in the laboratory can be used. A recombinant protein may be used as the reverse transcriptase.

Next, quantitative PCR is carried out using the template DNA (2) of the standardization factor obtained by the reverse transcription reaction of the standardization factor. In quantitative PCR, it is preferable to use the following two types of primer B2 and primer B3.
- Primer B2: a forward primer that specifically binds to the template DNA (2).
- Primer B3: a reverse primer that specifically binds to the template DNA (2). By carrying out quantitative PCR using these primers B2 and B3, the expression level of the standardization factor can be measured from the template DNA (2) of the standardization factor.

The DNA polymerase used for quantitative PCR is not particularly limited. Any DNA polymerase commonly used in the laboratory can be used. A recombinant protein may be used as the DNA polymerase.

The primer B1, the primer B2, and the primer B3 can be prepared by common nucleic acid synthesis and nucleotide synthesis. For example, the primers can be obtained from a company that undertakes contract synthesis of nucleic acids. In addition, TaqMan Advanced MicroRNA Assay can also be used as a commercially available kit for detecting the standardization factor. Therefore, a person skilled in the art can obtain the primer B1, the primer B2, and the primer B3 by a conventional method without undue burden, and therefore can examine the expression level of the standardization factor.

For example, the catalog number of the TaqMan Advanced MicroRNA Assay containing the following primer B11, primer B21, and primer B31 is A25576, and its Assay ID is 478925_mir.
- Primer B11: a looped RT primer that specifically binds to hsa-miR-4669.
- Primer B21: a forward primer that specifically binds to the template DNA (2) of hsa-miR-4669.
- Primer B31: a reverse primer that specifically binds to the template DNA (2) of hsa-miR-4669.

For example, the catalog number of the TaqMan Advanced MicroRNA Assay containing the following primer B12, primer B22, and primer B32 is A25576, and its Assay ID is 480284_mir.
- Primer B 12: a looped RT primer that specifically binds to hsa-miR-6756-5p.
- Primer B22: a forward primer that specifically binds to the template DNA (2) of hsa-miR-6756-5p.
- Primer B32: a reverse primer that specifically binds to the template DNA (2) of hsa-miR-6756-5p.

Next, in the method for assisting diagnosis of colorectal cancer of the present invention, the expression level of the colorectal cancer diagnostic marker is compared with the reference value. It is possible to determine whether or not a test object suffers from colorectal cancer by comparing the expression level of the colorectal cancer diagnostic marker with the reference value.

In a case where the expression level of the colorectal cancer diagnostic marker in the sample is relatively high with respect to the expression level of the colorectal cancer diagnostic marker in a healthy object, it can be determined that the test object suffers from colorectal cancer.

As the reference value, in a case where it is equal to or higher than this value, a reference value indicating suspected colorectal cancer can be considered. In a case of setting the reference value, the expression level of the colorectal cancer diagnostic marker in a healthy object may be referred to. Usually, the reference value is the expression level of the colorectal cancer diagnostic marker in a healthy object.

In a case where the expression level of the colorectal cancer diagnostic marker obtained by the measurement is higher than the reference value, it is determined that a test object suffers from colorectal cancer. On the other hand, in a case where the expression level of the colorectal cancer diagnostic marker obtained by the measurement is lower than the reference value, it is determined that a test object does not suffer from colorectal cancer.

The reference value used for determination can be appropriately set according to conditions such as the age and sex of the test object, the type of colorectal cancer diagnostic marker to be used, the collection method, and the type of specimen material.

In a case of determining the reference value, it is preferable to standardize an expression level of a colorectal cancer diagnostic marker in a healthy object using miRNA in a sample collected from a healthy subject as a standardization factor, and then use a standardized value of the expression level of the colorectal cancer diagnostic marker in the healthy object as the reference value.

By using the standardized value of the expression level of the colorectal cancer diagnostic marker in the healthy object as the reference value, it becomes easier to more accurately determine the relative magnitude of the expression level of the colorectal cancer diagnostic marker in a colorectal cancer test, and therefore it is possible to determine the presence or absence of colorectal cancer with higher accuracy.

The colorectal cancer diagnostic marker in the healthy object can be measured in the same manner as in the above-mentioned method (1). In addition, the expression level of the standardization factor in a healthy object can be measured in the same manner as in the above-mentioned method (2). Then, the reference value can be set as 2^{-ΔCt'} based on the following ΔCt'.
-ΔCt': a value obtained by subtracting the cutoff value of the standardization factor in the healthy object from the cutoff value of the colorectal cancer diagnostic marker in the healthy object.

As described above, in the method for assisting diagnosis of colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for assisting diagnosis of colorectal cancer of the present invention, the test accuracy, test sensitivity, and specificity in determining the presence or absence of colorectal cancer are excellent even in a case where the degree of progression of colorectal cancer is stage 0 or stage I.

### <Method for evaluating possibility of suffering from colorectal cancer>

In the method for evaluating the possibility of suffering from colorectal cancer of the present invention, an expression level of the colorectal cancer diagnostic marker of the present invention in a sample collected from a test object is measured, and the expression level is compared with a reference value.

The method for evaluating the possibility of suffering from colorectal cancer of the present invention can potentially provide information for making a decision for diagnosing whether or not a test object suffers from colorectal cancer. A diagnosis of colorectal cancer may be made based on the information for making a decision.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for evaluating the possibility of suffering from colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for evaluating the possibility of suffering from colorectal cancer of the present invention, the possibility of suffering from colorectal cancer can be evaluated with high accuracy even in a case where the degree of progression of colorectal cancer is stage 0 or stage I.

### <Method for diagnosis of colorectal cancer>

In the method for diagnosis of colorectal cancer of the present invention, an expression level of the colorectal cancer diagnostic marker of the present invention in a sample collected from a test object is measured, and the expression level is compared with a reference value.

The method for diagnosis of colorectal cancer of the present invention can provide information for making a decision for diagnosing whether or not a test object suffers from colorectal cancer. A diagnosis of colorectal cancer may be made based on the information for making a decision.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for diagnosis of colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for diagnosis of colorectal cancer of the present invention, it is possible to provide powerful information for making a decision for diagnosing colorectal cancer with high accuracy even in a case where the degree of progression of colorectal cancer is stage 0 or stage I.

### <Method for testing colorectal cancer>

In the method for testing colorectal cancer of the present invention, an expression level of the colorectal cancer diagnostic marker of the present invention in a sample collected from a test object is measured, and the expression level is compared with a reference value.

The method for testing colorectal cancer of the present invention can provide information for making a decision that assists in diagnosing whether or not a test object suffers from colorectal cancer. A diagnosis of colorectal cancer may be made based on the information for making a decision.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for testing colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for testing colorectal cancer of the present invention, it is possible to provide powerful information for making a decision for diagnosing colorectal cancer with high accuracy even in a case where the degree of progression of colorectal cancer is stage 0 or stage I.

### <Method for examining possibility of suffering from colorectal cancer>

In the method for examining the possibility of suffering from colorectal cancer of the present invention, an expression level of the colorectal cancer diagnostic marker of the present invention in a sample collected from a test object is measured, and the expression level is compared with a reference value.

The method for examining the possibility of suffering from colorectal cancer of the present invention can potentially provide information for making a decision for diagnosing whether or not a test object suffers from colorectal cancer. A diagnosis of colorectal cancer may be made based on the information for making a decision.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for examining the possibility of suffering from colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for examining the possibility of suffering from colorectal cancer of the present invention, the possibility that a test object suffers from colorectal cancer can be examined with high accuracy even in a case where the degree of progression of colorectal cancer is stage 0 or stage I.

<Method for collecting data for diagnosis of colorectal cancer>

In the method for collecting data for diagnosis of colorectal cancer of the present invention, an expression level of the colorectal cancer diagnostic marker of the present invention in a sample collected from a test object is measured.

The details and preferred aspect of the measurement of the expression level of the colorectal cancer diagnostic marker can be the same as described in <Method for assisting diagnosis of colorectal cancer>.

According to the method for collecting data for diagnosis of colorectal cancer of the present invention, information for diagnosing colorectal cancer with high accuracy can be obtained.

The information obtained by the method for collecting data for diagnosis of colorectal cancer of the present invention can be used for comparison with the reference value. As described above, the information is useful for providing information for making a decision for diagnosing colorectal cancer.

The specific contents of the reference value, and the details and preferred aspect of comparison of the expression level of the colorectal cancer diagnostic marker with the reference value can be the same as described in <Method for assisting diagnosis of colorectal cancer>.

As shown in Examples described later, according to the colorectal cancer diagnostic marker of the present invention, it is possible to separate an early stage 0 or stage I colorectal cancer patient from a healthy object with high accuracy.

Therefore, according to the method for collecting data for diagnosis of colorectal cancer of the present invention, in order to measure an expression level of a colorectal cancer diagnostic marker, it is possible to obtain powerful information for diagnosing colorectal cancer with high accuracy even in a case where the degree of progression of colorectal cancer is stage 0 or stage I.

### <In vitro method for assisting diagnosis of colorectal cancer>

In the in vitro method for assisting diagnosis of colorectal cancer of the present invention, an expression level of the colorectal cancer diagnostic marker of the present invention in a sample collected from a test object is measured.

The details and preferred aspect of the measurement of the expression level of the colorectal cancer diagnostic marker can be the same as described in <Method for assisting diagnosis of colorectal cancer>.

According to the in vitro method for assisting diagnosis of colorectal cancer of the present invention, information for diagnosing colorectal cancer with high accuracy can be obtained.

The information obtained by the in vitro method for assisting diagnosis of colorectal cancer of the present invention can be used for comparison with the reference value. As described above, the information is useful for providing information for making a decision for diagnosing colorectal cancer.

The specific contents of the reference value, and the details and preferred aspect of comparison of the expression level of the colorectal cancer diagnostic marker with the reference value can be the same as described in <Method for assisting diagnosis of colorectal cancer>.

As shown in Examples described later, according to the colorectal cancer diagnostic marker of the present invention, it is possible to separate an early stage 0 or stage I colorectal cancer patient from a healthy object with high accuracy.

Therefore, according to the in vitro method for assisting diagnosis of colorectal cancer of the present invention, in order to measure an expression level of a colorectal cancer diagnostic marker, it is possible to obtain powerful information for diagnosing colorectal cancer with high accuracy even in a case where the degree of progression of colorectal cancer is stage 0 or stage I.

### <Method for diagnosing colorectal cancer>

In the method for diagnosing colorectal cancer of the present invention, an expression level of the colorectal cancer diagnostic marker of the present invention in a sample collected from a test object is measured, and the expression level is compared with a reference value.

The method for diagnosing colorectal cancer of the present invention is a method for diagnosing whether or not a test object suffers from colorectal cancer.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for diagnosing colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for diagnosing colorectal cancer of the present invention, colorectal cancer can be diagnosed with high accuracy even in a case where the degree of progression of colorectal cancer is stage 0 or stage I.

### <Method for determining colorectal cancer>

In the method for determining colorectal cancer of the present invention, an expression level of the colorectal cancer diagnostic marker of the present invention in a sample collected from a test object is measured, and the expression level is compared with a reference value.

The method for determining colorectal cancer of the present invention is a method for determining whether or not a test object suffers from colorectal cancer.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for determining colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for determining colorectal cancer of the present invention, colorectal cancer can be determined with high accuracy even in a case where the degree of progression of colorectal cancer is stage 0 or stage I.

### <Method for examining colorectal cancer>

In the method for examining colorectal cancer of the present invention, an expression level of the colorectal cancer diagnostic marker of the present invention in a sample collected from a test object is measured, and the expression level is compared with a reference value.

The method for examining colorectal cancer of the present invention is a method for examining whether or not a test object suffers from colorectal cancer.

The details and preferred aspect of the measurement of the expression level of the colorectal cancer diagnostic marker can be the same as described in <Method for assisting diagnosis of colorectal cancer>.

The specific contents of the reference value, and the details and preferred aspect of comparison of the expression level of the colorectal cancer diagnostic marker with the reference value can be the same as described in <Method for assisting diagnosis of colorectal cancer>.

In the method for examining colorectal cancer of the present invention, the expression level of the colorectal cancer diagnostic marker of the present invention is compared with the reference value. Then, as shown in Examples described later, according to the colorectal cancer diagnostic marker of the present invention, it is possible to separate an early stage 0 or stage I colorectal cancer patient from a healthy object with high accuracy.

Therefore, according to the method for examining colorectal cancer of the present invention, it is possible to examine with high accuracy whether or not a test object suffers from colorectal cancer even in a case where the degree of progression of colorectal cancer is stage 0 or stage I.

### <Colorectal cancer diagnostic kit>

The colorectal cancer diagnostic kit of the present invention is a kit used for examining the presence or absence of colorectal cancer. The colorectal cancer diagnostic kit of the present invention includes a primer that is specific to the colorectal cancer diagnostic marker of the present invention.

Examples of the primer in the colorectal cancer diagnostic kit of the present invention include the primer A1, the primer A2, and the primer A3 described in <Method for assisting diagnosis of colorectal cancer>. The primer in the colorectal cancer diagnostic kit can form a complementary pair specifically with the colorectal cancer diagnostic marker of the present invention.

The details and preferred aspects of the primer A1, the primer A2, and the primer A3 can be the same as described in <Method for assisting diagnosis of colorectal cancer>.

The colorectal cancer diagnostic kit of the present invention may further include at least one selected from the group consisting of an extraction reagent for extracting microRNA, which is a colorectal cancer diagnostic marker, from a sample, a cDNA synthesis reagent for synthesizing cDNA of microRNA, a sample collection container, a primer that is specific to miRNA as a standardization factor, a reverse transcriptase, and an instruction manual.

Examples of the extraction reagent include common RNA extraction reagents. Specific examples of commercially available RNA extraction reagents include miRNeasy Serum/Plasma Kit (available from Qiagen, USA). However, the extraction reagent is not limited to these examples.

The cDNA synthesis reagent is a reagent for synthesizing cDNA of microRNA, which is a colorectal cancer diagnostic marker. Examples of commercially available cDNA synthesis reagents include TaqMan Advanced MicroRNA cDNA Synthesis Kit (available from Thermo Fisher Scientific, USA). However, the cDNA synthesis reagent is not limited to such an example.

The cDNA synthesis reagent may be used for the synthesis of the cDNA of miRNA which is used as a standardization factor.

The sample collection container is not particularly limited as long as it can accommodate a body fluid of a test object. For example, the sample collection container may be a common urinalysis container in a case where the body fluid is urine.

Examples of the primer for standardization include the primer B 1, the primer B2, and the primer B3 described in <Method for assisting diagnosis of colorectal cancer>. The primer for standardization can form a complementary pair specifically with miRNA as a standardization factor.

The details and preferred aspects of the primer B1 and the primer B2 can be the same as described in <Method for assisting diagnosis of colorectal cancer>.

Reverse transcriptase and DNA polymerase are reagents for quantifying microRNA, which is a colorectal cancer diagnostic marker, by RT-PCR. The method (1) described in <Method for assisting diagnosis of colorectal cancer> can be used for quantification of the colorectal cancer diagnostic marker. The reverse transcriptase is not particularly limited as long as the colorectal cancer diagnostic marker of the present invention can be reverse transcribed and the colorectal cancer diagnostic marker can be quantified.

The reverse transcriptase may be used for reverse transcription and quantification of miRNA used as the standardization factor. The reverse transcriptase is not particularly limited as long as it can be used for ordinary quantitative PCR.

The instruction manual in the colorectal cancer diagnostic kit of the present invention describes how to use the colorectal cancer diagnostic kit of the present invention.

The instruction manual may further contain information such as precautions for use, troubleshooting, and contact information. The information contained in the instruction manual is not limited to these examples. As described above, the instruction manual may be provided with at least enough information to obtain the effect of the present invention, and the items described therein are not particularly limited.

The method of using the colorectal cancer diagnostic kit of the present invention can be the same as described in <Method for assisting diagnosis of colorectal cancer>. For example, in a method of using the colorectal cancer diagnostic kit of the present invention, quantitative PCR is carried out using the primer in the colorectal cancer diagnostic kit of the present invention to quantify microRNA, which is a colorectal cancer diagnostic marker, and the measured value is compared with a reference value. However, the method of using the colorectal cancer diagnostic kit of the present invention is not limited to such an example.

### <Method for treating colorectal cancer>

In the method for treating colorectal cancer of the present invention, a test object is diagnosed using at least one or more selected from the group consisting of the method for assisting diagnosis of colorectal cancer of the present invention, the method for evaluating the possibility of suffering from colorectal cancer of the present invention, the method for diagnosis of colorectal cancer of the present invention, the method for testing colorectal cancer of the present invention, the method for examining the possibility of suffering from colorectal cancer of the present invention, the method for collecting data for diagnosis of colorectal cancer of the present invention, the in vitro method for assisting diagnosis of colorectal cancer of the present invention, the colorectal cancer diagnostic kit of the present invention, the method for diagnosing colorectal cancer of the present invention, the method for determining colorectal cancer of the present invention, and the method for examining colorectal cancer of the present invention, each of which is described hereinbefore.

In one aspect of the method for treating colorectal cancer of the present invention, a therapeutic agent for colorectal cancer is administered to a test object in a case where the test object is diagnosed as suffering from colorectal cancer.

In one aspect of the method for treating colorectal cancer of the present invention, a surgical procedure is carried out on a test object in a case where the test object is diagnosed as suffering from colorectal cancer.

In one aspect of the method for treating colorectal cancer of the present invention, at least one microRNA selected from a group consisting of hsa-miR-129-1-3p, hsa-miR-566, and hsa-miR-598-5p of a test object is inhibited.

In the method for treating colorectal cancer of the present invention, the diagnosis of a test object may be a determination of the presence or absence of colorectal cancer in the test object, or may be an examination of the presence or absence of colorectal cancer in the test object.

The therapeutic agent for colorectal cancer is not particularly limited. The therapeutic agent for colorectal cancer is preferably a drug whose potency and drug efficacy have been clinically confirmed.

The therapeutic agent for colorectal cancer may be the therapeutic agent for colorectal cancer of the present invention described later, or may be an anticancer agent, or may be a molecular targeted therapeutic agent. However, the therapeutic agent for colorectal cancer is not limited to these examples.

Examples of the anticancer agent include fluorouracil, a tegafur/uracil combination drug, a tegafur/gimeracil/oteracil potassium combination drug, capecitabine, irinotecan, oxaliplatin, and trifluridine/tipiracil hydrochloride. However, the anticancer agent is not limited to these examples. Even an anticancer agent that is not commercially available on the filing date of the present application and may be commercially available in the future may be used in the method for treating colorectal cancer of the present invention.

Examples of the molecular targeted therapeutic agent include cetuximab, panitumumab, bevacizumab, ramucirumab, aflibercept, and regorafenib. However, the molecular targeted therapeutic agent is not limited to these examples. Even a molecular targeted therapeutic agent that is not commercially available on the filing date of the present application and may be commercially available in the future may be used in the method for treating colorectal cancer of the present invention.

In a case where the test object is a non-human organism, the therapeutic agent for colorectal cancer may be a drug substance in the research stage, a drug substance in the development stage, or a drug substance in the clinical trial stage.

The method of administering the drug is not particularly limited. Usually, oral administration, intravenous drip, or the like is selected. However, the method of administering the drug is not limited to these examples.

The surgical procedure carried out on the test object is not particularly limited. Usually, endoscopic surgery, surgical resection, radiation therapy, or the like is selected. However, the surgical procedure carried out on the test object is not limited to these examples. Even a surgical procedure or treatment method that is not common on the filing date of the present application and may be carried out in the future may be used in the method for treating colorectal cancer of the present invention.

In the method for treating colorectal cancer of the present invention, at least one or more selected from the group consisting of the method for assisting diagnosis of colorectal cancer of the present invention, the method for evaluating the possibility of suffering from colorectal cancer of the present invention, the method for diagnosis of colorectal cancer of the present invention, the method for testing colorectal cancer of the present invention, the method for examining the possibility of suffering from colorectal cancer of the present invention, the method for collecting data for diagnosis of colorectal cancer of the present invention, the in vitro method for assisting diagnosis of colorectal cancer of the present invention, the colorectal cancer diagnostic kit of the present invention, the method for diagnosing colorectal cancer of the present invention, the method for determining colorectal cancer of the present invention, and the method for examining colorectal cancer of the present invention may be used, each of which is described hereinbefore, are used.

Therefore, according to the method for treating colorectal cancer of the present invention, the test object can be treated based on a highly accurate diagnostic result. Therefore, it is possible to prevent unnecessary treatments, reduce the burden on the patient, and reduce medical costs.

### <Therapeutic agent for colorectal cancer>

The therapeutic agent for colorectal cancer of the present invention contains an inhibitor of at least one microRNA selected from a group consisting of hsa-miR-129-1-3p, hsa-miR-566, and hsa-miR-598-5p.

Examples of the inhibitors of hsa-miR-129-1-3p, hsa-miR-566, and hsa-miR-598-5p include miRNA inhibitors. The miRNA inhibitor is an antisense nucleotide of microRNA. The miRNA inhibitor binds to microRNA serving as a sense strand to inhibit the function of the microRNA.

A commercially available kit can be used as the miRNA inhibitor. Examples of such a commercially available kit include mirVana miRNA inhibitor (available from Thermo Fisher Scientific, USA). For example, the catalog number of the mirVana miRNA inhibitor containing a miRNA inhibitor of hsa-miR-129-1-3p is 4464084, and its Assay ID is MH12962. In addition, the catalog number of the mirVana miRNA inhibitor containing a miRNA inhibitor of hsa-miR-566 is 4464084, and its Assay ID is MH11490.

In addition, RNAi reagents can be mentioned as inhibitors of hsa-miR-129-1-3p, hsa-miR-566, and hsa-miR-598-5p. However, the inhibitor is not limited to these examples. Any molecule capable of inhibiting the expression of hsa-miR-129-1-3p, hsa-miR-566, and hsa-miR-598-5p, even though it is an inhibitor that is not common on the filing date of the present application, may be used in the therapeutic agent for colorectal cancer of the present invention.

### (Mechanism of action)

The therapeutic agent for colorectal cancer of the present invention described above contains an inhibitor of at least one microRNA selected from a group consisting of hsa-miR-129-1-3p, hsa-miR-566, and hsa-miR-598-5p. As shown in Examples described later, inhibition of the expression of at least one microRNA selected from a group consisting of hsa-miR-129-1-3p, hsa-miR-566, and hsa-miR-598-5p results in a reduction in the cell motility of colorectal cancer cells. Therefore, it has been suggested that inhibitors of each microRNA of hsa-miR-129-1-3p, hsa-miR-566, and hsa-miR-598-5p may be useful as therapeutic agent candidates for colorectal cancer.

Based on the above suggestion, according to the therapeutic agent for colorectal cancer of the present invention, there is a possibility that a therapeutic effect on colorectal cancer can be obtained. In addition, it can be said that the therapeutic agent for colorectal cancer of the present invention containing an inhibitor of at least one microRNA selected from a group consisting of hsa-miR-129-1-3p, hsa-miR-566, and hsa-miR-598-5p is an agent for suppressing the motility of colorectal cancer cells. Similarly, it can be said that the method for treating colorectal cancer with an aspect that inhibits at least one microRNA selected from a group consisting of hsa-miR-129-1-3p, hsa-miR-566, and hsa-miR-598-5p of a test object is a method for suppressing the motility of colorectal cancer cells.

### [Examples]

Hereinafter, the present invention will be described in more detail by way of examples, but the present invention is not limited by the following description.

### <Explanation of abbreviations>

qRT-PCR: quantitative RT-PCR
median: median value
IQR: interquartile range
Healthy: healthy subject
CRC: colorectal cancer patient
Stage 0/I CRC: early stage 0 or stage I colorectal cancer patient
n: number of specimen materials
P value: p-value
AUC: area under the ROC curve
95% CI: 95% confidence interval

In the table, "E" means a power of 10. For example, "1.90E-04" means 1.90 × 10⁻⁴.

### <Construction of cohort>

First, from a total of 522 cases of a cohort consisting of 299 healthy subjects and 223 colorectal cancer patients, 415 cases randomly matched by age and gender were extracted, and the cohort of 415 cases was randomly classified into the following three cohorts: cohort 1, cohort 2, and cohort 3.
Cohort 1: 9 cases consisting of 6 healthy subjects and 3 colorectal cancer patients.
Cohort 2: 280 cases consisting of 140 healthy subjects and 140 colorectal cancer patients.
Cohort 3: 126 cases consisting of 63 healthy subjects and 63 colorectal cancer patients.

Here, the total number of healthy subjects in the cohort 2 and the cohort 3 is 203. In addition, among the colorectal cancer patients in the cohort 2 and the cohort 3, the total number of cases with a degree of progression of stage 0 or stage I was 64.

Therefore, the following cohort 4 was further constructed, and the detection sensitivity, accuracy, and specificity of early-stage colorectal cancer were examined by analysis of the cohort 4.

Cohort 4: 267 cases consisting of 203 healthy subjects and 64 stage 0 or stage I colorectal cancer patients.

### <Extraction of RNA>

In each cohort, RNA in a sample collected from urine was extracted.

200 µL of urine that had been cryopreserved at -80°C immediately after collection was used as a sample. Using miRNeasy Serum/Plasma Kit (available from Qiagen, USA), miRNA in the sample was extracted according to the conditions described in the attached protocol.

### <Synthesis of cDNA>

cDNA was synthesized using the extracted miRNA as a template. Using TaqMan Advanced MicroRNA cDNA Synthesis Kit (available from Thermo Fisher Scientific, USA), cDNA was synthesized according to the conditions described in the attached protocol.

### <qRT-PCR>

qRT-PCR was carried out using the synthesized cDNA. TaqMan Advanced MicroRNA Assay and 7500 Fast real-time PCR system (available from Thermo Fisher Scientific, USA) were used for qRT-PCR.

The thermal cycle and reaction conditions of qRT-PCR are shown in Table 1, and the list of Assay ID and target miRNA and each base sequence are shown in Table 2.

**[Table 1]**

| | Temperature [°C] | Time [sec] | Number of cycles [times] |
|---|---|---|---|
| Activation of enzyme | 95 | 20 | 1 |
| Heat denaturation | 95 | 3 | 40 |
| Annealing and elongation reactions | 60 | 30 | |

**[Table 2]**

| Target miRNA | Assay ID | miRNA sequence |
|---|---|---|
| hsa-miR-129-1-3p | 480873_mir | 5'-AAGCCCUUACCCCAAAAAGUAU-3' |
| hsa-miR-4669 | 478925_mir | 5'-UGUGUCCGGGAAGUGGAGGAGG-3' |
| hsa-miR-566 | 479373_mir | 5'-GGGCGCCUGUGAUCCCAAC-3' |
| hsa-miR-598-5p | 479080_mir | 5'-GCGGUGAUCCCGAUGGUGUGAGC-3 ' |
| hsa-miR-6756-5p | 480284_mir | 5'-AGGGUGGGGCUGGAGGUGGGGCU-3' |

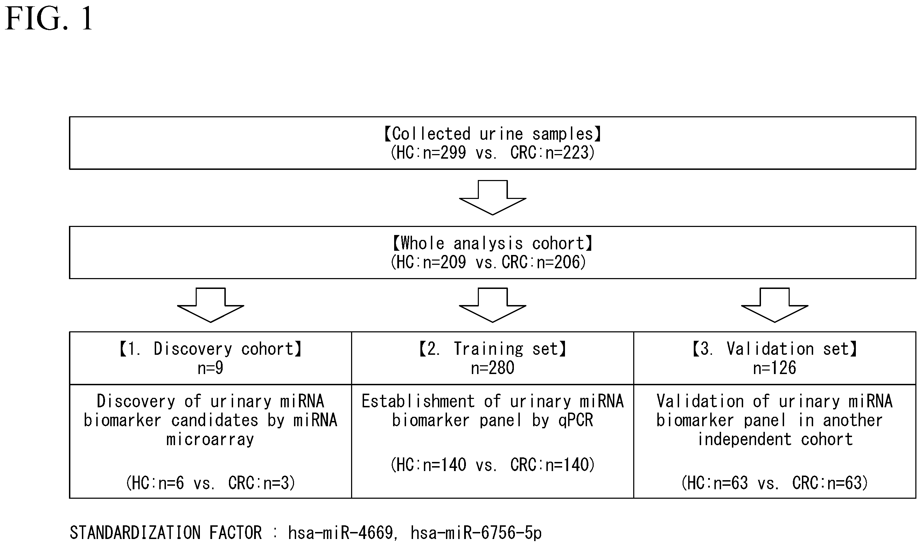

Fig. 1 is a flow chart showing an outline of verification of the present example.

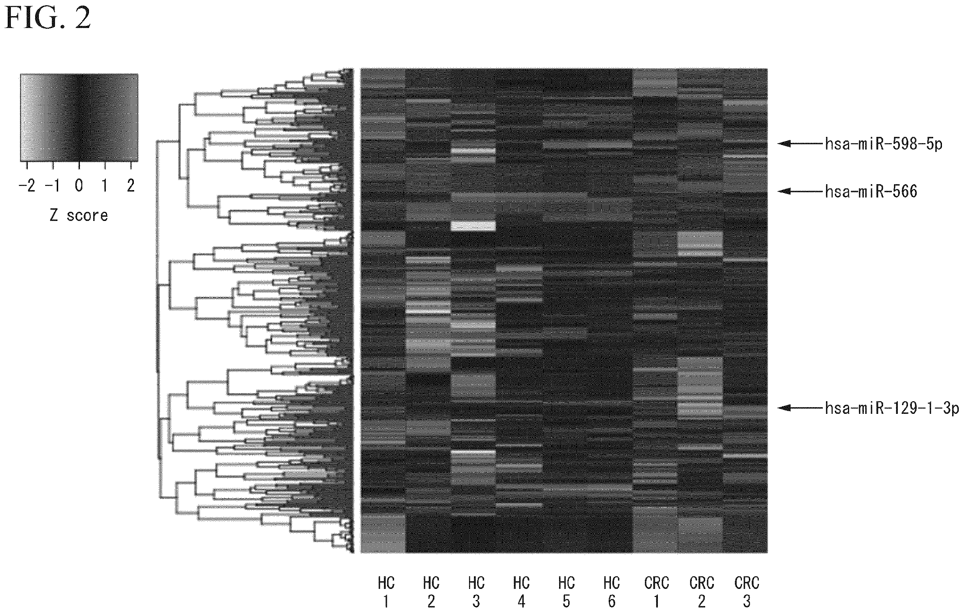

In the cohort 1, miRNA array analysis was carried out to select or identify multiple miRNAs abnormally expressed in the urine of colorectal cancer patients (see Fig. 2). The cohort 1 is a comprehensive analysis cohort (Discovery cohort in Fig. 1).

In the cohort 2, the expression levels of each of the three miRNAs serving as the colorectal cancer diagnostic marker of the present invention, among the miRNAs identified by the miRNA array analysis in the cohort 1, were measured by the qRT-PCR method. Significant colorectal cancer diagnostic markers were extracted by multivariate analysis of measurement results, and a genetic diagnosis panel was constructed. The cohort 2 is a training cohort (Training set in Fig. 2).

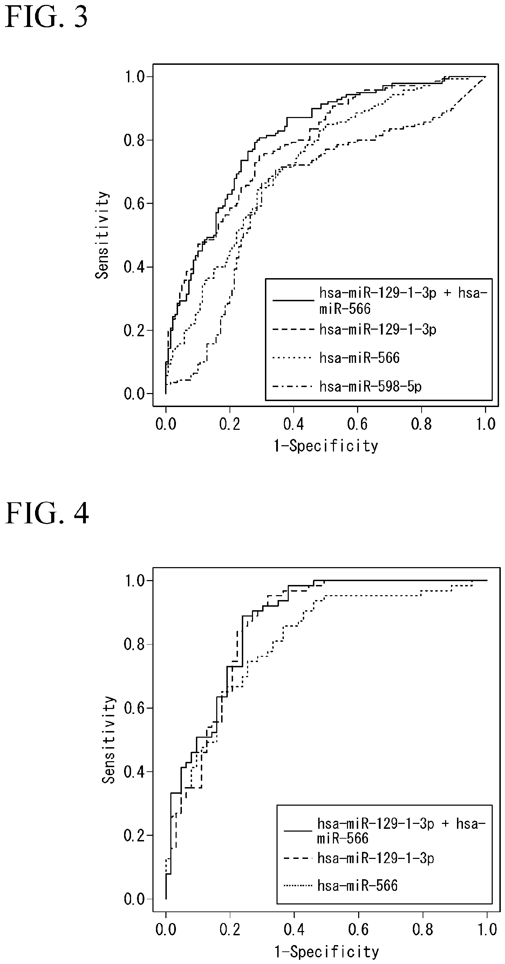

In the cohort 3, the accuracy of the genetic diagnosis panel constructed in the cohort 2 was verified. The cohort 3 is a validation cohort (Validation set in Fig. 3).

In the cohort 4, the detection accuracy of early-stage colorectal cancer was mainly analyzed.

Hereinafter, the specific contents and analysis results of the analysis of the cohort 1, the cohort 2, the cohort 3, and the cohort 4 carried out in the present example will be described in order.

### <Analysis of cohort 1>

Using the miRNAs extracted in the cohort 1, the analysis was carried out with SurePrint miRNA microarray (available from Agilent, USA). GeneSpring GX (available from Agilent) was used for analysis of data.

As a result of miRNA array analysis, 11 types of miRNAs that were significantly increased or decreased in the urine of colorectal cancer patients were identified.

### <Analysis of cohort 2>

11 types of miRNAs selected in the cohort 1 were measured by the qRT-PCR method. Table 3 shows the expression levels of each miRNA measured for three types of miRNAs, hsa-miR-129-1-3p, hsa-miR-566, and hsa-miR-598-5p, among the 11 types of miRNAs.

**[Table 3]**

| | | 2^{-ΔCt} | |
|---|---|---|---|
| | | Healthy (n = 140) | CRC (n = 140) |
| hsa-miR-129-1-3p | median | 5.40E-04 | 1.50E-03 |
| | IQR | 1.90E-4 to 1.01E-3 | 8.20E-4 to 3.26E-3 |
| hsa-miR-566 | median | 0.050 | 0.184 |
| | IQR | 0.029 to 0.163 | 0.071 to 0.438 |
| hsa-miR-598-5p | median | 0.122 | 0.273 |
| | IQR | 0.070 to 0.266 | 0.130 to 0.402 |

As shown in Table 3, in any of hsa-miR-129-1-3p, hsa-miR-566, and hsa-miR-598-5p, the value of 2^{-ΔCt}, that is, the measured value of the expression level of each of these genes was larger in colorectal cancer patients (CRC) than in healthy subjects (Healthy).

In the cohort 2, univariate analysis and multivariate analysis were carried out on the measurement results of the expression levels of each of the three types of microRNAs, hsa-miR-129-1-3p, hsa-miR-566 and hsa-miR-598-5p to verify the significant difference between healthy subjects and colorectal cancer patients. The results are shown in Table 4 and Fig. 3.

Fig. 3 is a view showing ROC curves of individual colorectal cancer diagnostic markers in the cohort 2. In Fig. 3, "1-Specificity" is plotted on the horizontal axis and "Sensitivity" is plotted on the vertical axis.

**[Table 4]**

| | Univariate analysis | Multivariate analysis | |
|---|---|---|---|
| | P value | Odds ratio (95% CI) | P value |
| hsa-miR-129-1-3p | < 0.001 | 5.59 (2.82 to 11.10) | < 0.001 |
| hsa-miR-566 | < 0.001 | 1.64 (1.09 to 2.45) | 0.017 |
| hsa-miR-598-5p | < 0.001 | - | - |

As shown in Table 4, the p-value was less than 0.001 even in the three types of microRNAs, hsa-miR-129-1-3p, hsa-miR-566, and hsa-miR-598-5p. From this, it could be confirmed that each of the microRNAs measured for the three types of hsa-miR-129-1-3p, hsa-miR-566 and hsa-miR-598-5p was significantly highly expressed in the urine of colorectal cancer patients as compared with healthy subjects.

In addition, from the results of multivariate analysis, hsa-miR-129-1-3p and hsa-miR-566 were extracted as independent colorectal cancer markers.

**[Table 5]**

| microRNA | AUC (95% CI) |
|---|---|
| Combination of hsa-miR-129-1-3p and hsa-miR-566 | 0.811 (0.762 to 0.861) |
| hsa-miR-129-1-3p alone | 0.790 (0.738 to 0.841) |
| hsa-miR-566 alone | 0.724 (0.666 to 0.783) |
| hsa-miR-598-5p alone | 0.640 (0.573 to 0.707) |

Table 5 shows the AUC of each ROC curve shown in Fig. 3 and 95% CI.

As shown in Table 5, it was confirmed that healthy subjects and colorectal cancer patients can be well separated by a genetic diagnosis panel prepared by using each of hsa-miR-129-1-3p, hsa-miR-566, and hsa-miR-598-5p as a colorectal cancer diagnostic marker.

As shown in Table 5, in the genetic diagnosis panel prepared by using a combination of hsa-miR-129-1-3p and hsa-miR-566 as the colorectal cancer diagnostic marker, the AUC of the ROC curve (Fig. 3) was 0.811, which was a particularly good result.

### <Analysis of cohort 3>

In the analysis of the cohort 3, the expression level of each microRNA was measured for two types of microRNAs, hsa-miR-129-1-3p and hsa-miR-566. Next, univariate analysis and multivariate analysis were carried out on the measurement results of the expression level of each microRNA to verify the significant difference between healthy subjects and colorectal cancer patients. The results are shown in Table 6.

Fig. 4 is a view showing ROC curves of individual colorectal cancer diagnostic markers in the cohort 3. Table 7 shows the AUC of each ROC curve shown in Fig. 4 and 95% CI. In Fig. 4, "1-Specificity" is plotted on the horizontal axis and "Sensitivity" is plotted on the vertical axis.

**[Table 6]**

| | | 2^{-ΔCt} | | Univariate analysis |
|---|---|---|---|---|
| | | Healthy (n = 63) | CRC (n = 63) | P value |
| hsa-miR-129-1-3p | median | 2.47E-04 | 1.41E-03 | < 0.001 |
| | IQR | 1.50E-4 to 7.94E-4 | 9.46E-4 to 2.18E-3 | |
| hsa-miR-566 | median | 0.040 | 0.222 | < 0.001 |
| | IQR | 0.017 to 0.105 | 0.100 to 0.526 | |

As shown in Table 6, also in the cohort 3, in both hsa-miR-129-1-3p and hsa-miR-566, the value of 2^{-ΔCt}, that is, the measured value of the expression level of each of these genes was larger in colorectal cancer patients than in healthy subjects.

In addition, as shown in Table 6, as a result of univariate analysis, the p-value was less than 0.001. From this, it could be confirmed that hsa-miR-129-1-3p and hsa-miR-566 were significantly highly expressed in the urine of colorectal cancer patients as compared with healthy subjects.

**[Table 7]**

| microRNA | AUC (95% CI) |
|---|---|
| Combination of hsa-miR-129-1-3p and hsa-miR-566 | 0.868 (0.806 to 0.931) |
| hsa-miR-129-1-3p alone | 0.856 (0.789 to 0.924) |
| hsa-miR-566 alone | 0.809 (0.733 to 0.885) |

As shown in Table 7, in the ROC curve (Fig. 4) of each microRNA, AUC exceeded 0.80 in each case. From this, it was found that, according to the genetic diagnosis panel prepared by using each microRNA as a colorectal cancer diagnostic marker, it is possible to separate healthy subjects and colorectal cancer patients with high accuracy.

In particular, in the genetic diagnosis panel prepared by using a combination of hsa-miR-129-1-3p and hsa-miR-566 as the colorectal cancer diagnostic marker, the AUC of the ROC curve (Fig. 4) was 0.868. From this, it was found that healthy subjects and colorectal cancer patients could be separated with particularly high accuracy, and therefore particularly good results were obtained.

The cohort 3 is a case group independent of the cohort 1 and the cohort 2. Therefore, the above analysis results of the cohort 3 suggest that hsa-miR-129-1-3p and hsa-miR-566 are useful as independent colorectal cancer diagnostic markers.

### <Analysis of cohort 4>

In the analysis of the cohort 4, the expression level of each microRNA was measured for hsa-miR-129-1-3p and hsa-miR-566. The results are shown in Table 8.

**[Table 8]**

| | | 2^{-ΔCt} | |
|---|---|---|---|
| | | Healthy (n = 203) | Stage 0/1 CRC (n = 64) |
| hsa-miR-129-1-3p | Median | 4.03E-04 | 1.52E-03 |
| | IQR | 1.62E-4 to 9.56E-4 | 8.86E-4 to 2.61E-3 |
| hsa-miR-566 | Median | 0.048 | 0.192 |
| | IQR | 0.026 to 0.140 | 0.079 to 0.431 |

As shown in Table 8, also in the cohort 4, in both hsa-miR-129-1-3p and hsa-miR-566, the value of 2^{-ΔCt}, that is, the measured value of the expression level of each of these genes was larger in colorectal cancer patients than in healthy subjects.

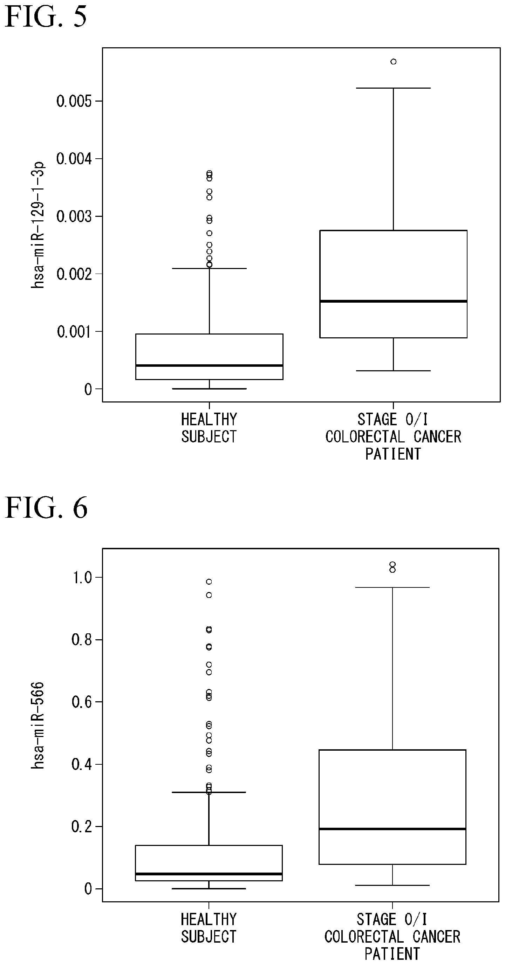

Next, univariate analysis and multivariate analysis were carried out on the measurement results of the expression level of each microRNA to verify the significant difference between healthy subjects and colorectal cancer patients. The results are shown in Fig. 5, Fig. 6, Fig. 7, and Fig. 9.

**[Table 9]**

| | Univariate analysis | Multivariate analysis | |
|---|---|---|---|
| | P value | Odds ratio (95% CI) | P value |
| hsa-miR-129-1-3p | < 0.001 | 2.85 (1.85 to 4.39) | < 0.001 |
| hsa-miR-566 | < 0.001 | 1.4 (1.00 to 1.97) | 0.051 |

As shown in Table 9, the p-value was less than 0.001 in both univariate analysis and multivariate analysis. From this, it could be confirmed that hsa-miR-129-1-3p and hsa-miR-566 were significantly highly expressed in the urine of colorectal cancer patients as compared with healthy subjects.

Fig. 5 is a view showing a comparison of expression levels of hsa-miR-129-1-3p in the cohort 4 between healthy subjects and stage 0 or stage I colorectal cancer patients.

Fig. 6 is a view showing a comparison of expression levels of hsa-miR-566 in the cohort 4 between healthy subjects and stage 0 or stage I colorectal cancer patients.

As shown in Fig. 5 and Fig. 6, in both cases of hsa-miR-129-1-3p and hsa-miR-566, each colorectal cancer diagnostic marker was significantly highly expressed in stage 0 or stage I colorectal cancer patients as compared with healthy subjects.

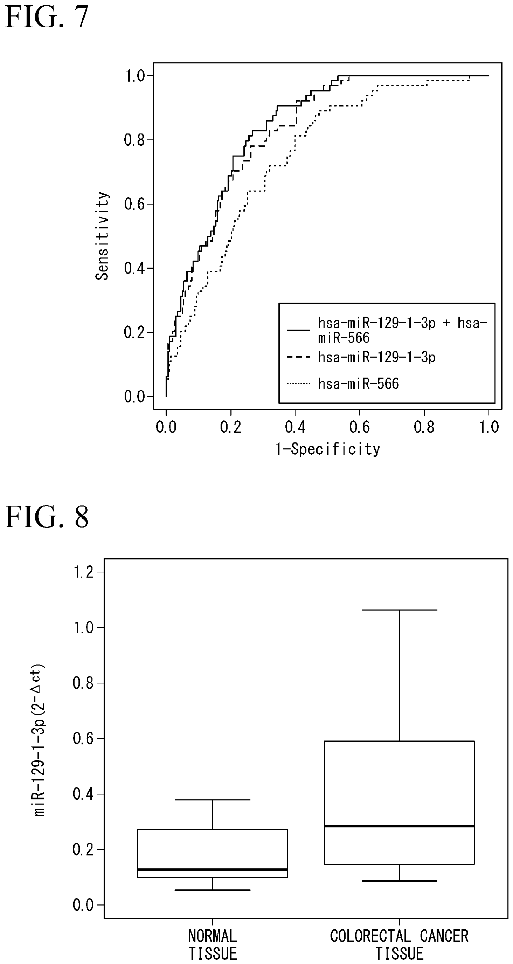

Fig. 7 is a view showing ROC curves of individual colorectal cancer diagnostic markers in the cohort 4. Table 10 shows the AUC of the ROC curve obtained in the cohort 4 and 95% CI. In Fig. 7, "1-Specificity" is plotted on the horizontal axis and "Sensitivity" is plotted on the vertical axis.

**[Table 10]**

| microRNA | AUC (95% CI) |
|---|---|
| Combination of hsa-miR-129-1-3p and hsa-miR-566 | 0.845 (0.798 to 0.893) |
| hsa-miR-129-1-3p alone | 0.832 (0.782 to 0.882) |
| hsa-miR-566 alone | 0.755 (0.692 to 0.819) |
| hsa-miR-598-5p alone | 0.698 (0.608 to 0.747) |

As shown in Table 10, the AUC was 0.845 in the ROC curve (Fig. 7) in which the microRNA was a combination of hsa-miR-129-1-3p and hsa-miR-566. Therefore, it was found that, according to the genetic diagnosis panel prepared by using the combination of hsa-miR-129-1-3p and hsa-miR-566 as the colorectal cancer diagnostic marker, healthy subjects and early-stage colorectal cancer patients can be separated with high accuracy even in a case of early-stage colorectal cancer patients with a degree of progression of stage 0 or stage I of colorectal cancer.

### <Example of cutoff value, sensitivity, and specificity in ROC curve>

An example of the cutoff value, sensitivity, and specificity in the ROC curve is shown below and in Table 11.

In a case where hsa-miR-598-5p was used alone as the colorectal cancer diagnostic marker, the cutoff value in the ROC curve was set to 0.177 or more. In this case, the sensitivity was 80.9% and the specificity was 67.9% (Table 11).

In a case where hsa-miR-566 was used alone as the colorectal cancer diagnostic marker, the cutoff value in the ROC curve was set to 0.054. In this case, the sensitivity was 87.5% and the specificity was 53.5% (Table 11).

In a case where hsa-miR-129-1-3p was used alone as the colorectal cancer diagnostic marker, the cutoff value in the ROC curve was set to 0.00499 or more. In this case, the sensitivity was 95.3% and the specificity was 54.2% (Table 11).

In a case where the combination of hsa-miR-129-1-3p and hsa-miR-566 was used as the colorectal cancer diagnostic marker, the cutoff value in the ROC curve was set to 0.152 or more. In this case, the sensitivity was 90.6% and the specificity was 65.5% (Table 11).

**[Table 11]**

| microRNA | Sensitivity [%] | Specificity [%] |
|---|---|---|
| hsa-miR-598-5p alone | 80.9 | 67.9 |
| hsa-miR-566 alone | 87.5 | 53.5 |
| hsa-miR-129-1-3p alone | 95.3 | 54.2 |
| Combination of hsa-miR-129-1-3p and miR-566 | 90.6 | 65.5 |

According to the genetic diagnosis panel prepared by using the combination of hsa-miR-129-1-3p and hsa-miR-566 as the colorectal cancer diagnostic marker, the AUC was 0.845 in distinguishing between healthy subjects and stage 0 or stage I colorectal cancer patients, which was particularly good (Table 10). Based on multivariate analysis, it was found that, according to the model formula using hsa-miR-129-1-3p and hsa-miR-566 expression, stage 0 or stage I colorectal cancer could be determined with sensitivity of 90.6% and specificity of 65.5% (Table 11).

### <Analysis using formalin-fixed paraffin embedding>

The expression levels of hsa-miR-129-1-3p and hsa-miR-566 were compared using formalin-fixed paraffin-embedded (FFPE) sections from 20 cases of colorectal cancer. The FFPE used was obtained by resection by endoscopy or surgical operation.

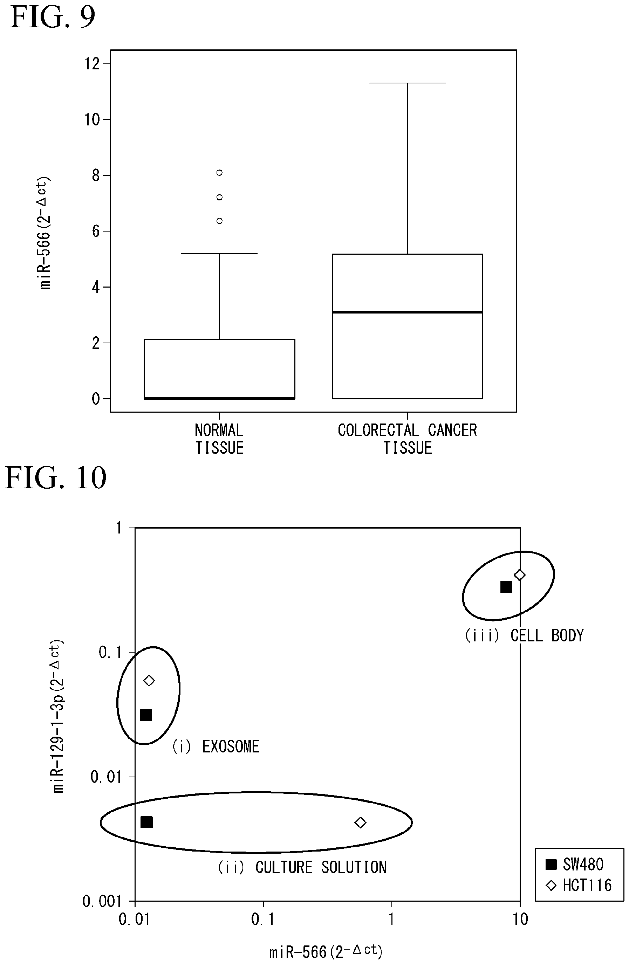

miRNA was extracted from both a colorectal cancer tissue and a surrounding normal tissue, and the expression levels of hsa-miR-129-1-3p and hsa-miR-566 were measured. In addition, univariate analysis was carried out based on the measurement results, and the p-value was calculated. The analysis results are shown in Table 12, and Fig. 8 and Fig. 9.

**[Table 12]**

| | | 2^{-ΔCt} | | Univariate analysis |
|---|---|---|---|---|
| | | Healthy (n = 20) | CRC (n = 20) | P value |
| hsa-miR-129-1-3p | Median | 0.128 | 0.284 | 0.009 |
| | IQR | 0.101 to 0.271 | 0.148 to 0.546 | |
| hsa-miR-566 | Median | 0 | 3.091 | 0.260 |
| | IQR | 0 to 1.785 | 0 to 5.184 | |

As shown in Table 12 and Fig. 8, hsa-miR-129-1-3p was significantly highly expressed in the colorectal cancer tissue as compared with the neighboring normal tissue.

On the other hand, as shown in Table 12 and Fig. 9, the same tendency as that of hsa-miR-129-1-3p was observed for hsa-miR-566, although there was no significant difference. The fact that no significant difference in the expression level of hsa-miR-566 was observed is considered to be due to the small number of specimen materials of 20.

### <Analysis using colorectal cancer cell line>

The expression levels of hsa-miR-129-1-3p and hsa-miR-566 were analyzed using a colorectal cancer cell line. Two types of colorectal cancer cell lines, that is, SW480 and HCT116 were used as the colorectal cancer cell line. Specifically, SW480 and HCT116 were cultured, and samples were collected from each of (i) exosomes in culture solution, (ii) culture solution, and (iii) cell body. The expression levels (2^{-ΔCt}) of hsa-miR-129-1-3p and hsa-miR-566 were measured for these samples. Fig. 10 shows a plot of the expression level (2^{-ΔCt}) of hsa-miR-129-1-3p on the vertical axis and the expression level (2^{-ΔCt}) of hsa-miR-566 on the horizontal axis.

In Fig. 10, (i) shows the expression level in exosomes, (ii) shows the expression level in the culture solution, and (iii) shows the expression level in cells.

As shown in Fig. 10, hsa-miR-129-1-3p and hsa-miR-566 were detectable from any of the samples (i) to (iii). In addition, hsa-miR-129-1-3p and hsa-miR-566 showed similar expression tendencies in any of the samples (i) to (iii).

From the above analysis results, it was suggested that hsa-miR-129-1-3p, hsa-miR-566, and hsa-miR-598-5p are useful as colorectal cancer diagnostic markers. In addition, in a case where the combination of hsa-miR-129-1-3p and hsa-miR-566 is used as the colorectal cancer diagnostic marker, detection can be made with high sensitivity even in a case of early-stage colorectal cancer, suggesting the possibility of separation from healthy objects.

### <Inhibition of colorectal cancer diagnostic marker>

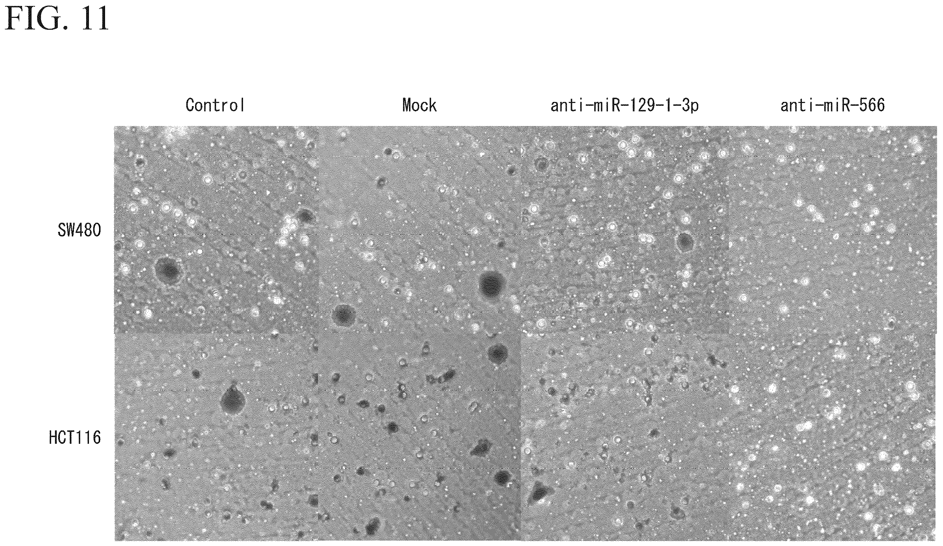

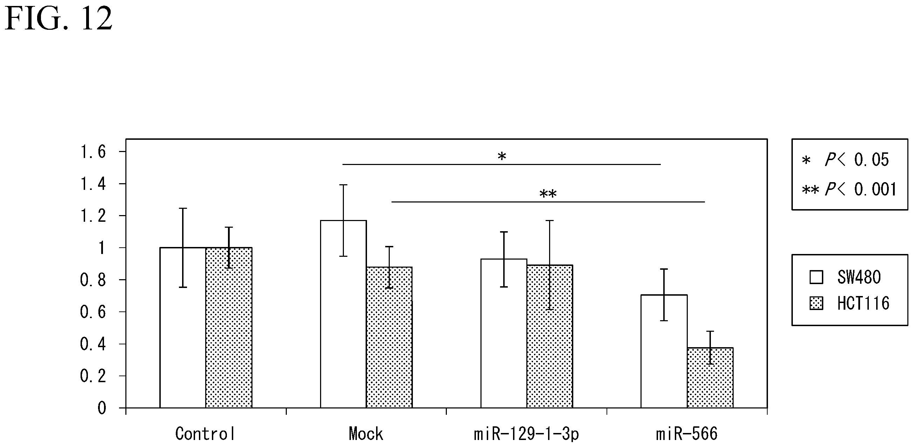

An inhibitor of hsa-miR-566 was administered to SW480 and HCT116. Here, mirVana miRNA inhibitor (catalog number: 4464084, Assay ID: MH11490, available from Thermo Fisher Scientific, USA) was used as the inhibitor of hsa-miR-566. In addition, Anti-miR miRNA Inhibitor Negative Control # 1 (catalog number: AM17010, available from Thermo Fisher Scientific, USA) was used as a negative control. Then, a migration assay was carried out to evaluate the cell motility of SW480 and HCT116. The results are shown in Fig. 11 and Fig. 12.

As shown in Fig. 11 and Fig. 12, the cell motility was significantly reduced in both SW480 and HCT116, in a case where the inhibitor of hsa-miR-566 was administered.

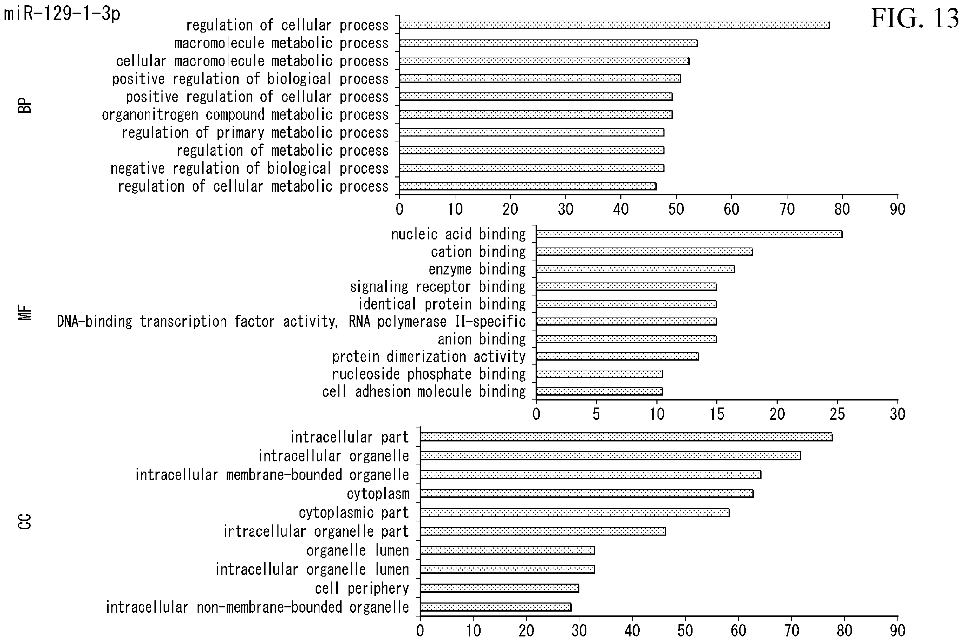

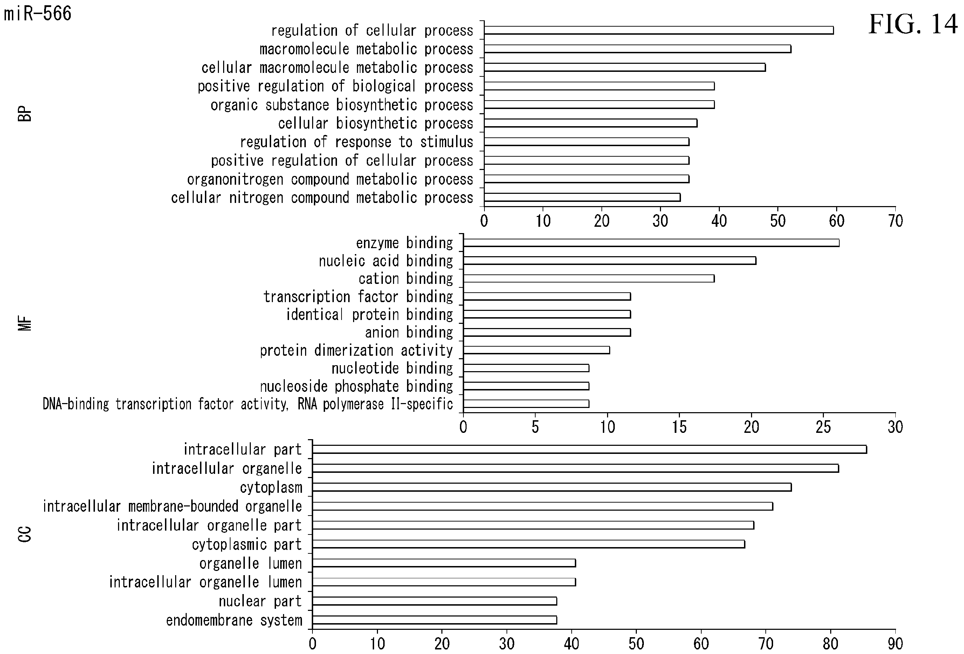

Here, gene ontology analysis was carried out using the data of miRNA target candidates of miRTarBase (http://mirtarbase.mbc.nctu.edu.tw/). The results are shown in Fig. 13 and Fig. 14.

The top 10 are shown in Fig. 13 and Fig. 14. Gene ontology terms common to both miRNAs are underlined. As shown in Fig. 13 and Fig. 14, hsa-miR-129-1-3p and hsa-miR-566 have many points in common with the functions of the target genes. Thus, it was suggested that hsa-miR-129-1-3p and hsa-miR-566 may have similar molecular biological functions to each other (Fig. 13 and Fig. 14).

Therefore, inhibition of hsa-miR-129-1-3p may also significantly reduce cell motility, so the inhibitor of hsa-miR-129-1-3p may also be useful as a therapeutic agent for colorectal cancer.

### [Industrial Applicability]

According to the present invention, the presence or absence of colorectal cancer can be determined with high accuracy, and early-stage colorectal cancer can be detected with high sensitivity.

## Claims

1. A colorectal cancer diagnostic marker, comprising:
at least one microRNA selected from a group consisting of hsa-miR-129-1-3p, hsa-miR-566, and hsa-miR-598-5p.

2. The colorectal cancer diagnostic marker according to Claim 1,
wherein the microRNA is a combination of hsa-miR-129-1-3p and hsa-miR-566.

3. The colorectal cancer diagnostic marker according to Claim 1 or 2,
wherein the colorectal cancer diagnostic marker is contained in a human body fluid.

4. A method for assisting diagnosis of colorectal cancer, comprising:
measuring an expression level of the colorectal cancer diagnostic marker according to any one of Claims 1 to 3 in a sample collected from a test object; and
comparing the expression level with a reference value.

5. The method according to Claim 4,
wherein the expression level is standardized using the following miRNA in the sample as a standardization factor in order to compare the expression level with the reference value,
miRNA: either or both of hsa-miR-4669 and hsa-miR-6756-5p.

6. The method according to Claim 4 or 5,
wherein a degree of progression of the colorectal cancer is stage 0 or stage I.

7. A method for collecting data for diagnosis of colorectal cancer, comprising:
measuring an expression level of the colorectal cancer diagnostic marker according to any one of Claims 1 to 3 in a sample collected from a test object.

8. The method according to Claim 7,
wherein, after measuring the expression level, the expression level is standardized using the following miRNA in the sample as a standardization factor,
miRNA: either or both of hsa-miR-4669 and hsa-miR-6756-5p.

9. The method according to Claim 7 or 8,
wherein a degree of progression of the colorectal cancer is stage 0 or stage I.

10. A colorectal cancer diagnostic kit for use in diagnosis of colorectal cancer, comprising:
a primer that is specific to the colorectal cancer diagnostic marker according to any one of Claims 1 to 3.

11. The colorectal cancer diagnostic kit according to Claim 10,
further comprising at least one selected from the group consisting of an extraction reagent for extracting the microRNA from a sample, a cDNA synthesis reagent for synthesizing cDNA of the microRNA, a sample collection container, a primer for standardization that is specific to miRNA as a standardization factor, a reverse transcriptase, a DNA polymerase, and an instruction manual.

12. The colorectal cancer diagnostic kit according to Claim 10 or 11,
wherein a degree of progression of the colorectal cancer is stage 0 or stage I.

13. A therapeutic agent for colorectal cancer, comprising:
an inhibitor of at least one microRNA selected from a group consisting of hsa-miR-129-1-3p, hsa-miR-566, and hsa-miR-598-5p.

14. A method for treating colorectal cancer, comprising:
inhibiting at least one microRNA selected from a group consisting of hsa-miR-129-1-3p, hsa-miR-566, and hsa-miR-598-5p of a test object.

15. A method for diagnosing colorectal cancer, comprising:
measuring an expression level of the colorectal cancer diagnostic marker according to any one of Claims 1 to 3 in a sample collected from a test object; and
comparing the expression level with a reference value.

16. The method for diagnosing colorectal cancer according to Claim 15,
wherein the expression level is standardized using miRNA in the sample as a standardization factor in order to compare the expression level with the reference value.

17. The method for diagnosing colorectal cancer according to Claim 16,
wherein the miRNA is either or both of hsa-miR-4669 and hsa-miR-6756-5p.

18. The method for diagnosing colorectal cancer according to Claim 15,
wherein a degree of progression of the colorectal cancer is stage 0 or stage I.

19. A method for treating colorectal cancer, comprising:
diagnosing a test object using the method for diagnosing colorectal cancer according to Claim 15; and
administering a therapeutic agent for colorectal cancer to the test object in a case where the test object is diagnosed as suffering from colorectal cancer.

20. A method for treating colorectal cancer, comprising:
diagnosing a test object using the method for diagnosing colorectal cancer according to Claim 15; and
carrying out a surgical procedure on the test object in a case where the test object is diagnosed as suffering from colorectal cancer.


### [Sequence Listing]

PC30356_sequence listing.txt

SEQUENCE LISTING

<110>  NAGOYA CITY UNIVERSITY

<120>  Biomarker for Colorectal Cancer Diagnosis, Method for Assisting
       Colorectal Cancer Diagnosis, Method for Collecting Data for
       Colorectal Cancer Diagnosis, Colorectal Cancer Diagnostic Kit

<130>  P1916

<160>  10

<170>  PatentIn version 3.5

<210>  1
<211>  22
<212>  RNA
<213>  Homo sapiens

<220>
<221>  misc_feature
<223>  Mature miRNA sequence of hsa-miR-129-1-3p

<400>  1
aagcccuuac cccaaaaagu au                                              22

<210>  2
<211>  19
<212>  RNA
<213>  Homo sapiens

<220>
<221>  misc_feature
<223>  Mature miRNA sequence of hsa-miR-566

<400>  2
gggcgccugu gaucccaac                                                  19

<210>  3
<211>  23
<212>  RNA
<213>  Homo sapiens

<220>
<221>  misc_feature
<223>  Mature miRNA sequence of hsa-miR-598-5p

<400>  3
gcggugaucc cgauggugug agc                                             23

<210>  4
<211>  22
<212>  RNA
<213>  Homo sapiens

<220>

<221>  misc_feature
<223>  Mature miRNA sequence of hsa-miR-4669

<400>  4
ugugucceggg aaguggagga gg                                              22

<210>  5
<211>  23
<212>  RNA
<213>  Homo sapiens

<220>
<221>  misc_feature
<223>  Mature miRNA sequence of hsa-miR-6756-5p

<400>  5
   aggguggggc uggagguggg gcu                                              23

<210>  6
<211>  72
<212>  RNA
<213>  Homo sapiens

<400>  6
ggaucuuuuu gcggucuggg cuugcuguuc cucucaacag uagucaggaa gcccuuaccc      60

caaaaaguau cu                                                          72

<210>  7
<211>  94
<212>  RNA
<213>  Homo sapiens

<400>  7
gcuaggcgug guggcgggcg ccugugaucc caacuacuca ggaggcuggg gcagcagaau      60

cgcuugaacc cgggaggcga agguugcagu gagc                                  94

<210>  8
<211>  97
<212>  RNA
<213>  Homo sapiens

<400>  8
gcuugaugau gcugcugaug cuggcgguga ucccgauggu gugagcugga aaugggugc      60

uacgucaucg uugucaucgu caucaucauc auccgag                               97

<210>  9
<211>  62
<212>  RNA
<213>  Homo sapiens

<400>  9
gccucccuuc acuuccuggc cauccaggca ucugugucug uguccgggaa guggaggagg      60

gc                                                                     62

<210>  10
<211>  63
<212>  RNA
<213>  Homo sapiens

<400>  10
acccuagggu ggggcuggag guggggcuga ggcugagucu uccuccccuu ccucccugcc      60

cag                                                                   63